# EUROPÄISCHE PATENTSCHRIFT

(11) **EP 0 879 032 B1**
(45) Veröffentlichungstag und Bekanntmachung des Hinweises auf die Patenterteilung: **15.01.2003**
(21) Anmeldenummer: 97909279.8
(22) Anmeldetag: 16.09.1997
(51) Int. Cl.: A61F 5/01

(54) **KNIEGELENKORTHESE**
KNEE HINGE
ORTHESE POUR L'ARTICULATION DU GENOU

(30) Priorität: 16.09.1996 DE 19637728
(43) Veröffentlichungstag der Anmeldung: 25.11.1998
(73) Patentinhaber: Bauerfeind Orthopädie GmbH & Co. KG, 47906 Kempen (DE)
(72) Erfinder: REINHARDT, Holger, D-47906 Kempen (DE); SCHEUERMANN, Rainer, D-24143 Kiel (DE); BAUERFEIND, Hans, Bruno, D-47906 Kempen (DE)
(74) Vertreter: Bardehle, Heinz, Dipl.-Ing.
(86) Internationale Anmeldenummer: EP9705072
(87) Internationale Veröffentlichungsnummer: WO98014144

(56) Entgegenhaltungen:
- CH-A- 108 380
- DE-A- 4 309 577
- US-A- 4 353 361
- US-A- 5 036 837
- US-A- 5 063 916
- US-A- 5 222 733

## Beschreibung

Die Erfindung bezieht sich auf eine Kniegelenkorthese mit Befestigungsmitteln für den Oberschenkel und den Unterschenkel, gemäß dem Oberbegriff des Anspruchs 1.

Eine derartige Kniegelenkorthese ist im Patent US 4,353,361 offenbart.

Eine andere Kniegelenkorthese ist in der europäischen Patentanmeldung 705 582 dargestellt und beschrieben. Die bekannte Kniegelenkorthese weist zu beiden Seiten des zu stützenden Knies zweiteilige Schienen auf, die mit ihren Enden jeweils in schalenartigen Befestigungmitteln enden, mit den die Orthese am Oberschenkel und am Unterschenkel angebracht ist. Die beiden Teile jeder Schiene sind über jeweils ein Gelenk verbunden, wobei das eine Gelenk (der inneren Schiene) aus einer Achse und das andere Gelenk (der äußeren Schiene) aus zwei gelenkig miteinander verbundenen Laschen bestehen, die sich überkreuzen und jeweils mit ihrem einen Ende an dem einen Befestigungsmittel und ihrem anderen Ende an dem anderen Befestigungsmittel angelenkt sind.

Das Prinzip der drehbeweglichen Verbindung von zweiteiligen Schienen einer Kniegelenksorthese geht auf die bekannte Erkenntnis zurück. daß im Kniegelenk eine Roll-Gleit-Bewegung erfolgt. Diese Bewegung wird in einer Veröffentlichung von A.Bähler "Die biomechanischen Grundlagen der Orthesenversorgung des Knies" Orthopädie-Technik 2/89, Seiten 52-59, erläutert. Dabei wird in Abbildung 6 auf Seite 54 dieser Veröffentlichung eine "Kompromißachse" nach Nietert dargestellt, die aufgrund von in der Veröffentlichung erläuterten Untersuchungen gefunden wird. Hierzu wird in der Einleitung der Veröffentlichung auf Seite 52, linke Spalte, Absatz 1, folgendes ausgeführt: "Die Ergebnisse zeigen, daß einachsige Schienen mit ca. 16 mm Achsrückverlagerung für mittelgroße Patienten, Doppelgelenkschienen mit Verzahnung, Vierachsgelenke sowie physiologische Gelenke mit wandernder Achse den Anforderungen noch am besten genügen."

In der Orthopädietechnik sind eine Vielzahl von Kniegelenkorthesen vorgeschlagen worden, die mehr oder minder die vorstehend erläuterten Erkenntnisse berücksichtigen. Abgesehen von komplizierten Kniegelenkorthesen mit einer Mehrzahl von Laschen- bzw. Hebelverbindungen, die mit einem erheblichen technischen Aufwand verbunden sind, werden in großem Umfang Orthesen verwendet, bei denen ihre beiden Schienenteile nur durch eine einzige Achse verbunden sind. Für die optimale Gestaltung einer derartigen Orthese kommt es entscheidend auf die Lage dieser Achse in Bezug auf das Kniegelenk an. In der EP-PS 393 081 ist eine Kniegelenkorthese beschrieben, bei der beidseitig des Knies jeweils ein einachsiges Drehgelenk vorgesehen ist, dessen Relativlage zu zwei Befestigungsmitteln am Ober- und Unterschenkel durch eine Verschiebung von an den Gelenken angebrachten Armen herbeigeführt wird, die mit mehreren Löchern zum wahlweisen Verbinden mit an den Befestigungsmitteln angebrachten Gegenstücken versehen sind. Durch Ausnutzung der Löcher können die Gelenke mehr oder minder an die Befestigungsmittel herangerückt werden. Hierbei handelt es sich offensichtlich um einen relativ aufwendigen Mechanismus, der zudem nur eine stufenweise Anpassung der Lage der Drehgelenke an die Befestigungsmittel ermöglicht.

Der Erfindung liegt die Aufgabe zugrunde, eine Kniegelenkorthese gemäß den eingangs angegebenen Merkmalen zu schaffen, die bei konstruktiv einfachem Aufbau eine praktisch automatische Anpassung der Lage ihrer Achse an die Kniebewegung des jeweiligen Patienten individuell ermöglicht. Erfindungsgemäß geschieht dies durch die technischen Merkmale des Kennzeichenden Teils des Anspruchs 1.

Je nach der für notwendig erkannten Intensität der Behandlung des Knies eines Patienten kann man die erfindungsgemäße Kniegelenkorthese mit nur einer zweiteiligen Schiene versehen, die dann entweder auf der Innen- oder Außenseite des Knies anzuordnen ist, oder es wird die Kniegelenksorthese mit zwei zweiteiligen Schienen auf beiden Seiten des Knies versehen, womit sich natürlich eine besonders stabile Führung des zu behandelnden Knies ergibt.

Aufgrund der exzentrischen Lagerung der Achse in dem Drehlager, das die beiden Schienenteile drehbeweglich miteinander verbindet, ergibt sich bei der Kniebewegung gegebenenfalls eine Druck- bzw. Zugkraft auf die Achse, wobei diese Kräfte sich auf das Drehlager übertragen und dieses gegenüber den beiden Schienenteilen so verdrehen, daß schließlich die Achse eine Lage einnimmt, in der die Achse weitgehend an den Bewegungsablauf des Kniegelenks angepaßt ist, d.h. in der die Lage der Achse mit der oben erwähnten "Kompromißachse" weitgehend übereinstimmt. Dabei ist von besonderem Vorteil, daß die Verschiebung der Achse in Anpassung an die Kniegelenkbewegung aufgrund der exzentrischen Lagerung der Achse längs eines Kreisbogens erfolgt, so daß diese Bewegung sowohl eine Komponente quer als auch längs zum Bein enthält. Hierdurch ergibt sich eine besonders gute Anpassungsmöglichkeit der Achslage an die jeweilige Kniegelenkbewegung im Sinne einer Verschiebung der Achse möglichst dicht an die "Kompromißachse" heran.

Diese Anpassung der Lage der Achse erfolgt durch die Kniegelenkbewegung des jeweiligen Patienten, der damit eine Anpassung individuell an die bei ihm gegebenen anatomischen Verhältnisse automatisch herbeiführt, gegebenenfalls mit unterschiedlicher Verschiebung der Achse auf der rechten und linken Seite der Kniegelenkorthese, ohne daß es hierzu eines Eingriffes im Sinne irgendeiner Manipulation bedarf.

Je nach der vom behandelnden Arzt für erforderlich angesehenen Therapie kann es sich darum handeln, die Verschiebung der Lage der Achse mit der Verdrehung des Drehlagers ständig bei der Bewegung des Kniegelenks zuzulassen oder für den Fall einer gewollten Fixierung der Achse diese in ihrer angepaßten Lage zu arretieren, so daß, ausgehend von dieser arretierten Lage, die Achse das Kniegelenk entsprechend führt, womit das Kniegelenk gegen Fehlbewegungen geschützt wird.

Um diesen letzteren Fall zu behandeln, versieht man das Drehlager zweckmäßig mit Arretierungsmitteln gegenüber der Schiene. Wenn dann durch die Kniegelenkbewegung die Achse ihre angepaßte Lage gefunden hat, wird die Arretierung des Drehlagers vorgenommen, so daß damit die Achslage festgelegt ist. Diese läßt sich mit lösbaren Arretierungsmitteln natürlich wieder aufheben, womit es möglich wird, eine im Rahmen einer längeren Therapie erforderlich werdende erneute Anpassung der Lage der Achse an unter Umständen geänderte Verhältnisse beim Patienten herbeizuführen, z.B. durch Verstärkung seiner Muskulatur aufgrund eines Trainings.

Für die sichere Halterung der zweiteiligen Schiene in der Kniegelenkorthese wird die Schiene zweckmäßig mit ihren beiden Teilen jeweils in eine Tasche am Befestigungsmittel für den Oberschenkel und für den Unterschenkel eingeschoben. Damit die Schiene nach ihrer Einbringung in die Taschen auch wieder herausgenommen werden kann, werden die Taschen zweckmäßig so ausgebildet, daß die eine Tasche durchgehend seitlich geschlossen und zum Drehgelenk hin geöffnet ist, während die andere Tasche eine in ihrer Längsrichtung verlaufende Öffnung zum Einführen und Herausnehmen der Schiene besitzt, die mit einer mittels eines Klettverschlusses verschließbaren Abdecklasche abgedeckt ist.

In den Figuren sind Ausführungsbeispiele der Erfindung dargestellt.
Es zeigen:
- Fig. 1: die Kniegelenkorthese von der Seite gesehen;
- Fig. 2: das Drehlager einer zweiteiligen Schiene der Drehgelenkorthese gemäß Figur 1 in vergrößerter Darstellung;
- Fig. 3: eine zweiteilige Schiene mit dem Drehlager im Schnitt und
- Fig. 4: die Kniegelenkorthese gemäß Fig. 1 mit einer zu öffnenden Tasche zum Einführen und Herausnehmen der Schiene.

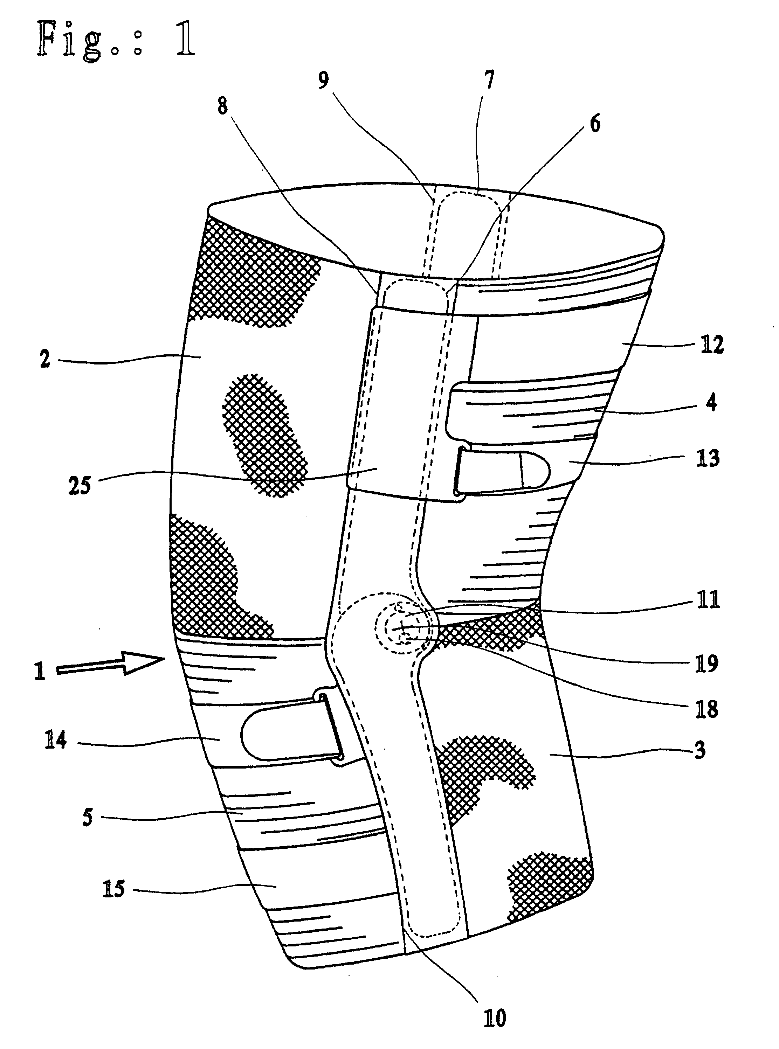

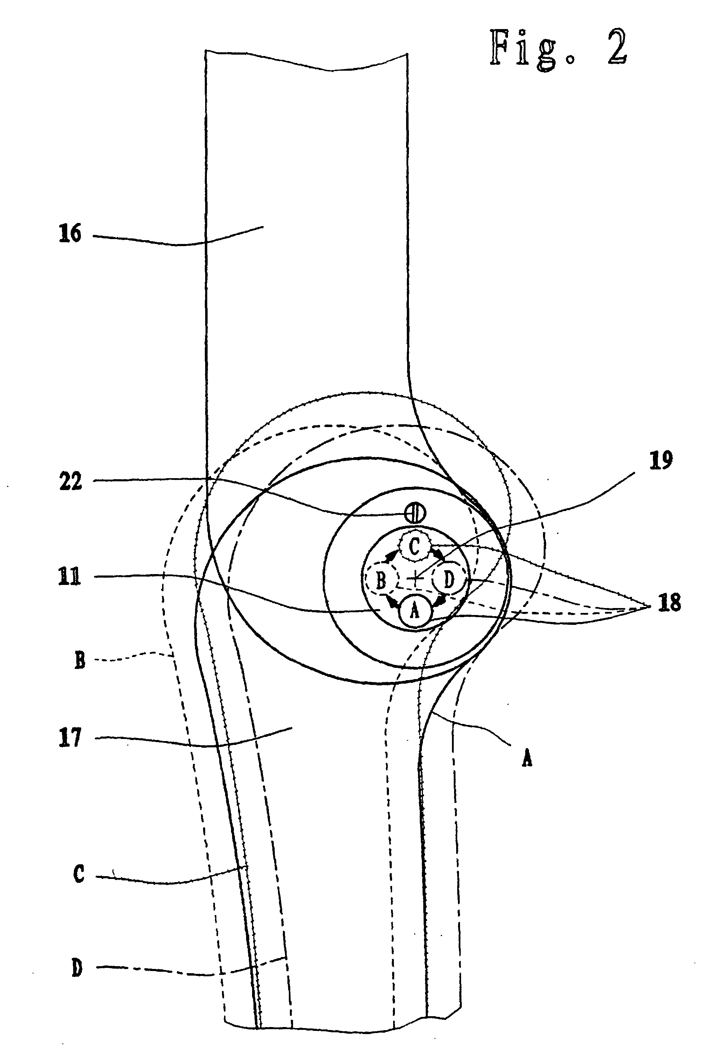

In Figur 1 ist die Kniegelenkorthese 1 dargestellt, die durch einen Schlauch gebildet ist und die beiden unelastischen Schlauchteile 2 und 3 sowie die elastischen Schlauchteile 4 und 5 enthält. Die unelastischen Schlauchteile 2 und 3 sind gewissermaßen diagonal angeordnet, was auch für die elastischen Schlauchteile 4 und 5 gilt. Im Bereich des Zusammenstoßes der unelastischen Schlauchteile 2/3 mit den elastischen Schlauchteilen 4/5 sind die zweiteiligen Schienen 6 und 7 eingezeichnet, die zu beiden Seiten der Kniegelenkorthese 1 angeordnet sind. Die beiden Teile jeder der beiden Schienen 6 und 7 sind in Taschen 8,9 und 10 eingeschoben und von diesen eng umschlossen, so daß beim Bewegen des Knies und damit der Schlauchteile 2/3 und 4/5 die beiden Teile jeder der Schienen 6 und 7 sich gegeneinander um die Achse 18 verdrehen. Bezüglich der Anordnung der Achse 18 wird auf die Erläuterungen zu Figur 2 verwiesen. Die elastischen Schlauchteile 4 und 5 der Kniegelenkorthese 1 sind durch die unelastischen Bänder 12, 13, 14 und 15 überbrückt, die an den unelastischen Schlauchteilen 2 und 3 befestigt sind und den elastischen Schlauchteilen 4 und 5 nach dem Anlegen an ein Knie die Elastizität in Umfangsrichtung nehmen, womit der Kniegelenkorthese 1 ein sicherer Sitz am Knie gegeben wird.

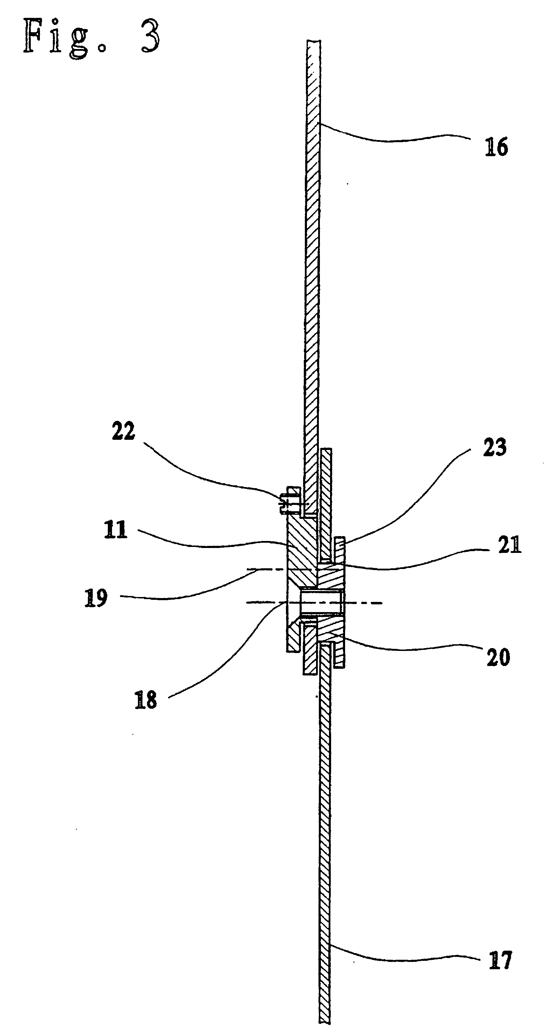

In Figur 2 ist eine der Schiene 6 aus Figur 1 entsprechende zweiteilige Schiene mit ihrem Oberteil 16 und ihrem Unterteil 17 dargestellt. Die Schiene 16/17 enthält das Drehlager 11, über das das Oberteil 16 mit dem Unterteil 17 verbunden ist. Das Drehlager 11 ist ein scheibenartiges Gebilde, das, wie die weiter unten erläuterte Figur 3 zeigt, in das Oberteil 16 verdrehbar eingefügt ist. Das Drehlager 11 enthält die exzentrische Achse 18, die außerdem in das Unterteil 17 eindringt und damit die Drehachse bildet, um die sich Oberteil und Unterteil gegeneinander verdrehen lassen. Aufgrund der Verdrehbarkeit des Drehlagers 11 ist die Möglichkeit gegeben, die Achse 18 in Bezug auf das Oberteil 16 auf einer Kreisbahn zu bewegen, die sich aufgrund der Exzentrizität der Achse 18 gegenüber der Mitte des Drehlagers 11 ergibt. Diese Mitte ist durch die strichpunktierte Linie 19 in Figur 3 angedeutet. In Figur 2 ist die Mitte des Drehlagers 11 durch das in seiner Mitte eingezeichnete Kreuz 19 dargestellt.

Aufgrund einer Verdrehung des Drehlagers 11 können sich für die Achse 18 die in Figur 2 durch die Buchstaben A,B,C,D angegebenen Positionen (und kontinuierlich alle Zwischenpositionen) ergeben, womit die Achse 18 jeweils eine entsprechende Lage einnimmt. Hieraus ergibt sich eine Relativlage von Oberteil 16 und Unterteil 17, die in der Figur 2 durch Linienführungen angedeutet ist, die mit den Umrandungen der Achslagen A,B,C und D übereinstimmen. Aufgrund dieser unterschiedlichen Linienführungen ist zu erkennen, daß in Bezug auf die jeweilige Linienführung die zugehörige Kniegelenkorthese 1 relativ zu dem Drehlager 11 verschoben ist, was in Bezug auf das von der Kniegelenkorthese 1 eingeschlossene Knie bedeutet, daß die Achse 18 in Bezug auf das eingeschlossene Knie entsprechend verschoben ist. Die Achse 18 läßt sich also mit dem Drehlager 11 relativ zu dem betreffenden Kniegelenk über den Bereich der die Achslagen A,B,C,D, enthaltenden Kreisbahn verschieben, womit für die Annäherung an die "Kompromißachse" eine weitgehende Anpassungsmöglichkeit geschaffen ist.

Aus der die Schiene 16/17 gemäß Figur 2 im Schnitt darstellenden Figur 3 ist ersichtlich, daß das Drehlager 11 an dem Oberteil 16 mittels einer Schraube gehalten ist, welch letztere gleichzeitig die Achse 18 für die gegenseitige Verdrehbarkeit von Oberteil 16 und 17 bildet. Die die Achse 18 bildende Schraube ist zentrisch in das Mutterstück 20 eingeschraubt, das in eine entsprechende Bohrung 21 des Unterteils 17 eingesetzt und am Unterteil 17 mittels des Flansches 23 festgehalten ist.

Aufgrund der vorstehend beschriebenen Gestaltung des Drehlagers 11 mit der Achse 18 und dem Mutterstück 20 ergibt sich eine exzentrische Lagerung der Achse 18 gegenüber der Mitte 19 des Drehlagers 11, die oben unter Bezugnahme auf die Achslagen A,B,C und D erläutert ist. Diese Anordnung erlaubt es, daß sich das Drehlager 11 leicht im Oberteil 16 drehen läßt, so daß bei an ein Knie angelegter Kniegelenkorthese 1 und Bewegung des Knies sich das Drehlager 11 in eine Lage verschiebt, in der die Achse 18 weitestgehend der "Kompromißachse" angenähert ist.

Um in der so erreichten günstigsten Lage der Achse 18 das Drehlager 11 gegenüber dem Oberteil 16 zu fixieren, ist die Madenschraube 22 vorgesehen, bei deren Anziehen diese gegen das Oberteil 16 drückt und damit das Drehlager 11 gegenüber dem Oberteil 16 fixiert. Damit ist gleichzeitig die Achse 18 gegenüber dem Oberteil 16 fixiert, womit sich für die Verdrehung von Oberteil 16 und Unterteil 17 gegeneinander eine nicht verschiebbare Achse ergibt.

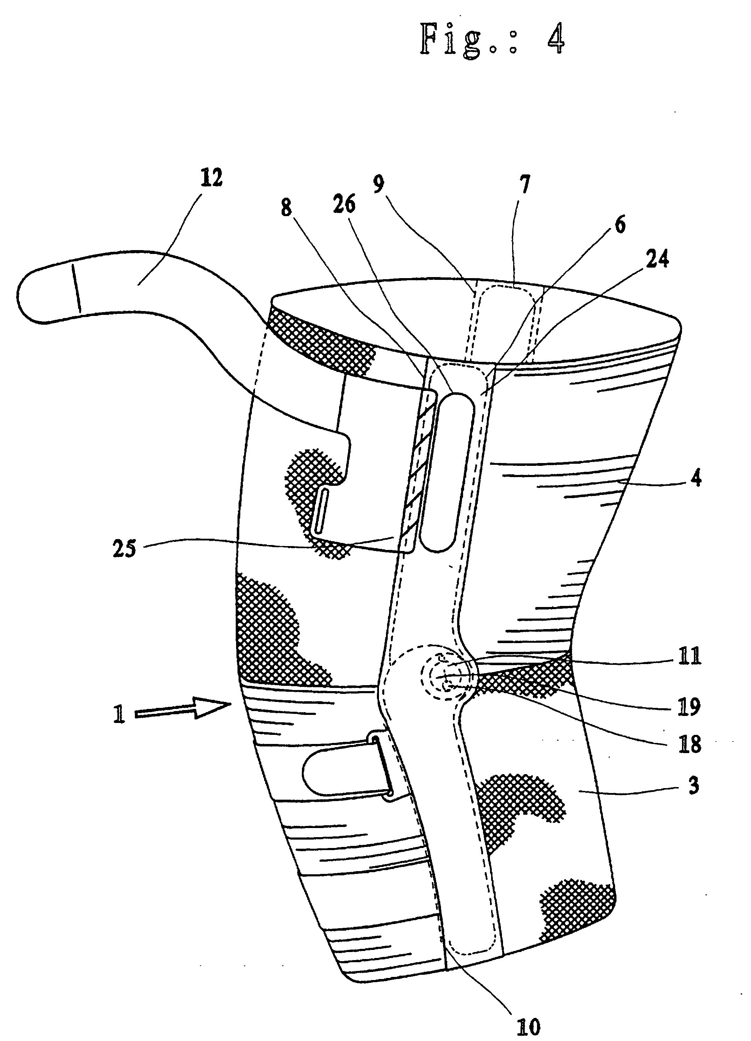

In der Figur 4 ist die Kniegelenkorthese 1 gemäß Figur 1 dargestellt, wobei zusätzlich eingezeichnet ist, wie die Schiene 6 (und damit auch die Schiene 7) in die Orthese eingesetzt und aus dieser herausgenommen werden kann. Das untere Teil der Schiene 6 ist in die auf das unelastische Schlauchteil 3 aufgesetzte Tasche eingesetzt. Für den oberen Teil der Schiene 6 ist die zu öffnende Tasche 24 vorgesehen, für den unteren Teil die Tasche 10. Die Tasche 24 weist die Öffnung 26 und die Abdecklasche 25 auf, die über die Tasche 24 in Richtung auf den elastischen Schlauchteil 4 gelegt werden und an diesem mittels eines Klettverschlusses befestigt werden kann. Damit ist die Schiene 6 in den Taschen 24 und 10 festgehalten und kann aus diesen nicht herausgleiten. Mit Öffnen der Abdecklasche 25 kann die Schiene 6 aus den beiden Taschen 24 und 10 herausgenommen werden, beispielsweise falls dies für eine Reinigung der Kniegelenkorthese 1 erforderlich ist. Das gleiche gilt für die Schiene 7 in der Tasche 9.

## Patentansprüche

1. Kniegelenkorthese (1) mit Befestigungsmitteln für den Oberschenkel und den Unterschenkel, die über mindestens eine zweiteilige Schiene (6, 7) miteinander verbunden sind, die ein gegenüber dem Kniegelenk in Anpassung an dessen Bewegungsablauf zurückversetztes Drehgelenk mit einer Achse (18) aufweist, die in einem Drehlager (11) exzentrisch gelagert ist, das beide Schienenteile (6, 7) drehbeweglich verbindet und durch seine Verdrehung die Lage der Achse (18) kontinuierlich verschiebt, wobei diese Verschiebung durch die Kniegelenkbewegung herbeigeführt wird, **dadurch gekennzeichnet, dass** die Verschiebung entlang einer Kreislinier (A,B,C,D) erfolgt und die Achse (18) in einer beliehigen Position entlang dieser Kreislinie (A,B,C,D) durch Arretierungsmittel (22) gegenüber der Schiene (6, 7) festlegbar ist.

2. Kniegelenkorthese nach Anspruch 1, **dadurch gekennzeichnet, dass** die Schiene (6, 7) mit ihren beiden Teilen jeweils in eine Tasche (24, 10) am Befestigungsmittel für den Oberschenkel und für den Unterschenkel eingeschoben ist

3. Kniegelenkorthese nach Anspruch 2, **dadurch gekennzeichnet, dass** die eine Tasche (10) durchgehend seitlich geschlossen und zum Drehgelenk hin geöffnet ist, während die andere Tasche (24) eine in ihrer Längsrichtung verlaufende Öffnung (26) zum Einführen und Herausnehmen der Schiene besitzt, die mit einer mittels eines Klettverschlusses verschließbaren Abdecklasche (25) abgedeckt ist.

## Revendications

1. Orthèse (1) destinée à l'articulation du genou, comportant des moyens de fixation pour la cuisse et la jambe qui sont reliés entre eux par l'intermédiaire d'au moins une éclisse (6, 7) en deux parties qui, pour l'adaptation au déroulement des mouvements de l'articulation du genou, comporte une articulation tournante qui est décalée vers l'arrière par rapport à l'articulation du genou avec un axe (18) qui est monté de façon excentrée dans un coussinet de pivotement (11), lequel relie en rotation les deux parties d'éclisse (6, 7) et déplace en permanence la position de l'axe (18) par sa rotation, ce déplacement étant provoqué par le mouvement de l'articulation du genou, **caractérisée en ce que** le déplacement est effectué le long d'une ligne circulaire (A, B, C, D), et **en ce que**, le long de cette ligne circulaire (A, B, C, D), l'axe (18) peut être immobilisé dans une position quelconque par rapport à l'éclisse (6,7) par des moyens de blocage (22).

2. Orthèse destinée à l'articulation du genou selon la revendication 1, **caractérisée en ce que** l'éclisse (6, 7) avec ses deux parties est insérée dans une poche (24, 10) prévue sur les moyens de fixation pour la cuisse et pour la jambe.

3. Orthèse destinée à l'articulation du genou selon la revendication 2, **caractérisée en ce que** l'une des poches (10) est fermée latéralement de bout en bout et ouverte vers l'articulation tournante, alors que l'autre poche (24) comporte une ouverture (26) s'étendant dans sa direction longitudinale pour l'insertion et le retrait de l'éclisse, qui est recouverte par une languette de recouvrement (25) pouvant être fermée par une fermeture velcro.

## Claims

1. Knee-joint orthesis (1) with fastening means for the thigh and the lower leg, which fastening means are connected to one another via at least one two-part rail (6, 7) having a pivot hinge which is offset rearwards in relation to the knee joint, to adapt to the pattern of movement of the latter, which pivot hinge has a pin (18) which is mounted eccentrically in a pivot bearing (11), which pivot bearing connects both rail parts (6, 7) in a rotationally movable manner and, by its turning movement, continuously displaces the position of the pin (18), this displacement being effected by the movement of the knee joint, **characterized in that** the displacement occurs along a circular line (A,B,C,D) and the pin (18) can be fixed in any desired position along this circular line (A,B,C,D) in relation to the rail (6, 7) using locking means (22).

2. Knee-joint orthesis according to Claim 1, **characterized in that** the rail (6, 7) is pushed with its two parts in each case into a pocket (24, 10) on the fastening means for the thigh and for the lower leg.

3. Knee-joint orthesis according to Claim 2, **characterized in that** one pocket (10) is closed all along its side and is open towards the pivot hinge, while the other pocket (24) has an opening (26), extending in its longitudinal direction, for the insertion and removal of the rail, and which is covered by a covering flap (25) which can be closed by means of a touch-and-close fastener.
